# EUROPEAN PATENT SPECIFICATION

(11) **EP 1 083 927 B1**
(45) Date of publication and mention of the grant of the patent: **26.05.2004**
(21) Application number: 98926210.0
(22) Date of filing: 03.06.1998
(51) Int. Cl.: A61K 39/395, C12Q 1/68, G01N 33/53, C12P 21/04, C12N 15/63, C12N 15/85, C12N 15/11, C07H 21/04

(54) **HUMAN MINOR VAULT PROTEIN p193**
MENSCHLICHES "MINOR VAULT" PROTEIN P193
PROTEINE VOUTE MINEURE HUMAINE p193

(43) Date of publication of application: 21.03.2001
(73) Proprietor: THE REGENTS OF THE UNIVERSITY OF CALIFORNIA, Oakland, California 94607-5200 (US)
(72) Inventor: ROME, Leonard, H., Tarzana, CA 91356 (US); KICKHOEFER, Valerie, A., Sherman Oaks, CA 92354 (US)
(74) Representative: Hall, Robert Leonard
(86) International application number: PCT/US1998/011348
(87) International publication number: WO 1999/062547

(56) References cited:
- KEDERSHA N.L. ET AL.: "Vaults. II. Ribonucleoprotein structures are highly conserved among higher and lower eukaryotes" J. CELL BIOL., vol. 110, April 1990 (1990-04), pages 895-901, XP002002541
- ROME L. ET AL.: "Unlocking vaults: organelles in search of a function" TRENDS CELL BIOL., vol. 1, August 1991 (1991-08) - September 1991 (1991-09), pages 47-50, XP002002540
- SCHROEIJERS A.B. ET AL.: "The Mr 193,000 vault protein is up-regulated in multidrug-resistant cancer cell lines" CANCER RES., vol. 60, no. 4, 15 February 2000 (2000-02-15), pages 1104-1110, XP001029820
- SIVA A. ET AL.: "Identification and characterization of the minor vault protein, p193" MOL. BIOL. CELL, vol. 9, November 1998 (1998-11), page 69A XP000982180
- KIRCKHOEFER V.A. ET AL.: "The 193-kD vault protein, VPARP, is a novel poly(ADP-ribose) polymerase" J. CELL BIOL., vol. 146, no. 5, 6 September 1999 (1999-09-06), pages 917-928, XP002180527
- KICKHOEFER V. et al., "Multidrug Resistant Cancer Cell Lines Contain Elevated Levels of Vaults", PROC. AMER. ASSOC. CANCER RES., March 1997, page 252, Abstract #1694, XP002911906
- SEBOLT-LEOPOLD J. et al., "Enhancement of Alkylating Agent Activity In Vitro by PD 128763, a Potent Poly(ADP-ribose) Synthetase Inhibitor", INT. J. RADIATION ONCOLOGY BIOL. PHYS., 1992, Vol. 22, pages 619-621, XP002911907
- KIM K. et al., "Tumor Suppressor Gene Expression During Normal and Pathologic Myocardial Growth", J. BIOL. CHEM., 09 September 1994, Vol. 269, No. 36, pages 22607-22613, XP002911908
- KICKHOEFER V. et al., "Vaults are Up-Regulated in Multidrug-Resistant Cancer Cell Lines", J. BIOL. CHEM., 10 April 1998, Vol. 273, No. 15, pages 8971-8974, XP002911909
- DATABASE GENBANK [Online] OHARA O. ET AL.: 'Human mRNA for KIAA0177 Gene', XP002907349 Retrieved from EMBL Database accession no. D79999

## Description

The present invention was made with government support under Grant No. GM 38097, awarded by the National Institutes of Health. The United States Government has certain rights in this invention.

### BACKGROUND

Cancer is a major cause of morbidity and mortality in the United States. Treatment of cancer generally includes chemotherapy, radiation therapy and surgery. Unfortunately, most cancers cannot be cured using chemotherapy because tumor cells tend to develop resistance to several chemotherapeutic agents over time. These cancers are referred to as "multidrug-resistant cancers" (MDR).

Overexpression of a number of proteins has been found to be associated with MDR cells lines, including P-glycoprotein (Pgp) and multidrug resistance-associated protein (MRP). These proteins appear to mediate drug resistance by acting as cytotoxic drug efflux pumps. However, many MDR cancer cell lines are known which are not associated with overexpression of either P-glycoprotein or multidrug resistance-associated protein.

More recently, a protein has been described that is overexpressed in MDR tumor cell lines which do not overexpress either P-glycoprotein or multidrug resistance-associated protein. This protein was originally named Lung Resistance-related Protein (LRP), referring to the cell line in which it was originally identified. However, once the cDNA for Lung Resistance-related Protein was isolated and the corresponding protein sequence elucidated, it was found that Lung Resistance-related Protein was human major vault protein, a previously known protein.

Vaults are large, barrel-shaped, multi-subunit, cytoplasmic, ribonucleoprotein organelles found in virtually all higher organisms and in most normal tissues. Mammalian vaults consist of three proteins having molecular weights of approximately 210, 193 and 104, and a small RNA in the relative molar ratios of 1:1:24:4 in rats. The most abundant of these, the 104 kDa protein, is termed major vault protein (MVP) and corresponds to the Lung Resistance-related Protein. The minor vault protein p193, however, has not yet been characterized.

Therefore, there remains a need for chemotherapeutic agents that will target multidrug-resistant cancers. Further, there remains a need to characterize the minor vault protein p193.

### SUMMARY

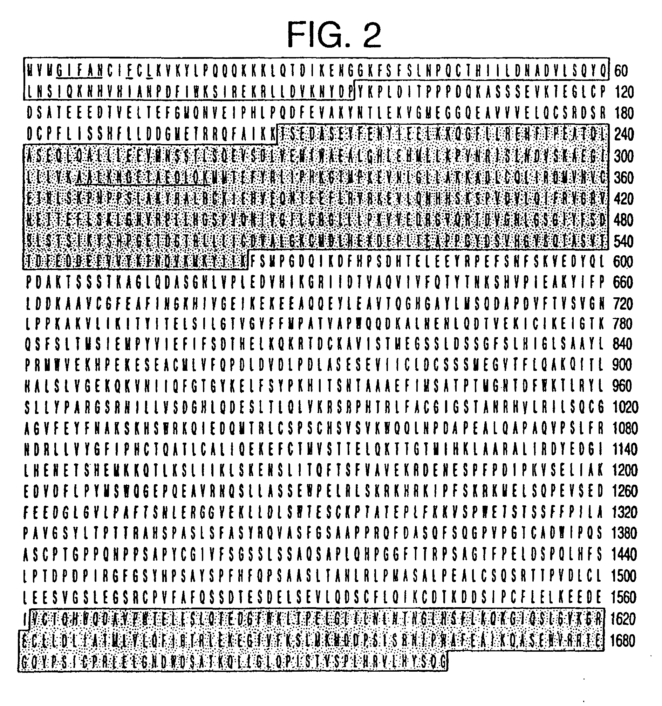

According to one embodiment of the present invention, there is provided a protein consisting essentially of purified isolated, or purified and isolated human minor vault protein p193. The protein can be recombinant and can have an amino acid sequence as set forth in Figure 2, SEQ ID NO:2. Further, the protein can be a protein recognized by a monoclonal antibody having affinity thereto.

According to another embodiment of the present invention, there is provided a polynucleotide molecule encoding a protein according to the present invention or its complementary strands. The molecule can be RNA or DNA, or can be another polynucleotide molecule.

According to another embodiment of the present invention, there is provided a vector containing a polynucleotide molecule according to the present invention or a prokaryotic or eukaryotic host cell stably transformed or transfected by the vector.

According to another embodiment of the present invention, there is provided a high affinity monoclonal antibody which immunoreacts with a protein according to the present invention. The antibody can have an Fc portion selected from the group consisting of the IgM class, the IgG class and the IgA class.

According to another embodiment of the present invention, there is provided a method of making a monoclonal antibody which immunoreacts with human minor vault protein p193 comprising the steps of, first, administering human minor vault protein p193 to a host in an amount sufficient to induce the production of antibodies to the human minor vault protein p193 from the antibody-producing cells. Then, the antibody-producing cells are recovered from the host. Next, cell hybrids are formed by fusing the antibody-producing cell to cells capable of substantially unlimited reproduction. Then, the hybrids are cultured. Further, the monoclonal antibodies are collected as a product of the hybrids. The cells capable of substantially unlimited reproduction can be myeloma cells.

According to another embodiment of the present invention, there is provided a method of making a protein according to the present invention comprising the steps of, first, culturing a microorganism transformed with a polynucleotide encoding human minor vault protein p193. Then, the human minor vault protein p193 is recovered.

According to another embodiment of the present invention, there is provided a method of diagnosing a patient with a multidrug-resistant cancer comprising the steps of, first, providing a sample of tissue or fluid from the patient. Then, the level of a substance selected from the group consisting of p193 protein, p193 DNA, p193 mRNA, a substantial portion of p193 protein, a substantial portion of p193 DNA, a substantial portion of p193 mRNA and a combination of one of the foregoing in the patient sample is determined. Next, the level of the substance is compared to a known range of levels for the substance in patients with multidrug-resistant cancers. A diagnosis of multidrug-resistant cancer is made when the level of the substance determined is within the range of levels for the substance in patients with multidrug-resistant cancers. The sample can be selected from the group consisting of bone marrow, cerebral spinal fluid, blood, tears, saliva and a biopsy specimen.

According to another embodiment of the present invention, there is provided a method of treating a patient with multidrug-resistant cancer comprising the steps of, first, diagnosing a patient with multidrug-resistant cancer according to the present invention, and then treating the patient. The treatment can comprise administering to the patient antibodies having an affinity for a substance selected from the group consisting of p193 protein and a polynucleotide encoding p193. The treatment can also comprise administering to the patient at least one antisense polynucleotide having an affinity for a polynucleotide encoding p193. The treatment can further comprise administering to the patient at least one drug that blocks NAD, such as PD128763 and 3-aminobenzamide.

According to another embodiment of the present invention, there is provided a method of catalyzing the hydrolysis of the nicotine moiety or of catalyzing the polymerisation of the ADP ribose group of at least one NAD molecule comprising contacting the NAD molecule with a protein comprising an amino acid sequence as set forth in SEQ ID NO:2.

### FIGURES

These and other features, aspects and advantages of the present invention will become better understood with regard to the following description, appended claims, and accompanying figures where: Figurel shows the complete sequence of cDNA encoding human minor vault protein p193, top strand, and its complementary strand; and
Figure 2 shows the complete amino acid sequence of human minor vault protein p193 indicating specific regions of function.

### DESCRIPTION

The present invention involves the elucidation of the amino acid sequence for human vault protein p193, as well as the DNA sequence encoding human vault protein p193. These sequences are then utilized in methods of diagnosing multidrug resistance cancer and in methods of treating multidrug resistance cancer.

### (1) Elucidation of the Human Minor Vault Protein p193 Amino Acid Sequence and the Nucleotide Sequence Encoding Human Minor Vault Protein p193:

The human minor vault protein p193 amino acid sequence and the nucleotide sequence encoding human minor vault protein p193 were elucidated as follows. First, human vault protein p193 was cloned using an interaction trap, two-hybrid system according to techniques known to those with skill in the art. See, for example, Golemis, et al., *Current Protocols in Mol. Biol.* 20.1.1-20.35 John Willey & Sons, 1997, incorporated by reference in its entirety. In summary, rat major vault protein, GenBank accession number U09870, having the 67 amino acids at the amino-terminal truncated was used as bait against a HeLa acid fusion cDNA library obtained from Roger Brent, Boston, MA, USA to search for proteins that interacted with rat major vault protein. The interacting proteins were identified by their ability to give rise to blue colonies on media containing galactose and X-gal, a color indicator substrate. The specificity of the interaction between the identified proteins and the rat major vault protein was verified by retransformation of the identified proteins with specific, rat major vault protein and nonspecific (lexA-bicoid) bait cDNAs. This technique identified the cDNA encoding the 193 kDa minor vault protein, SEQ ID NO: 1, by its interaction with the rat major vault protein.

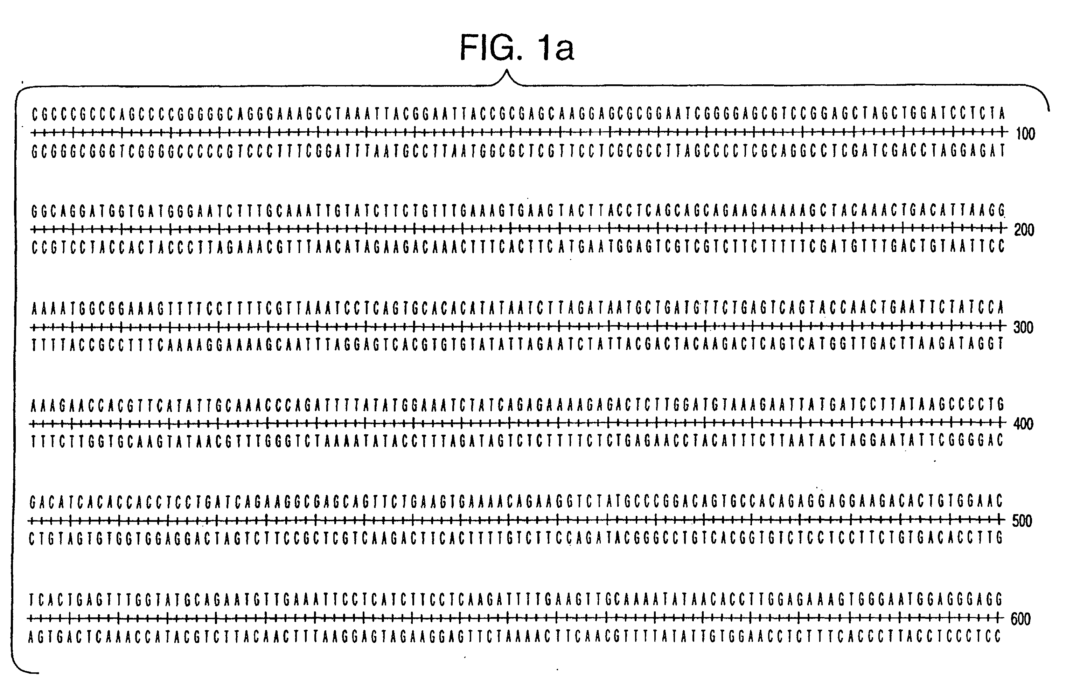

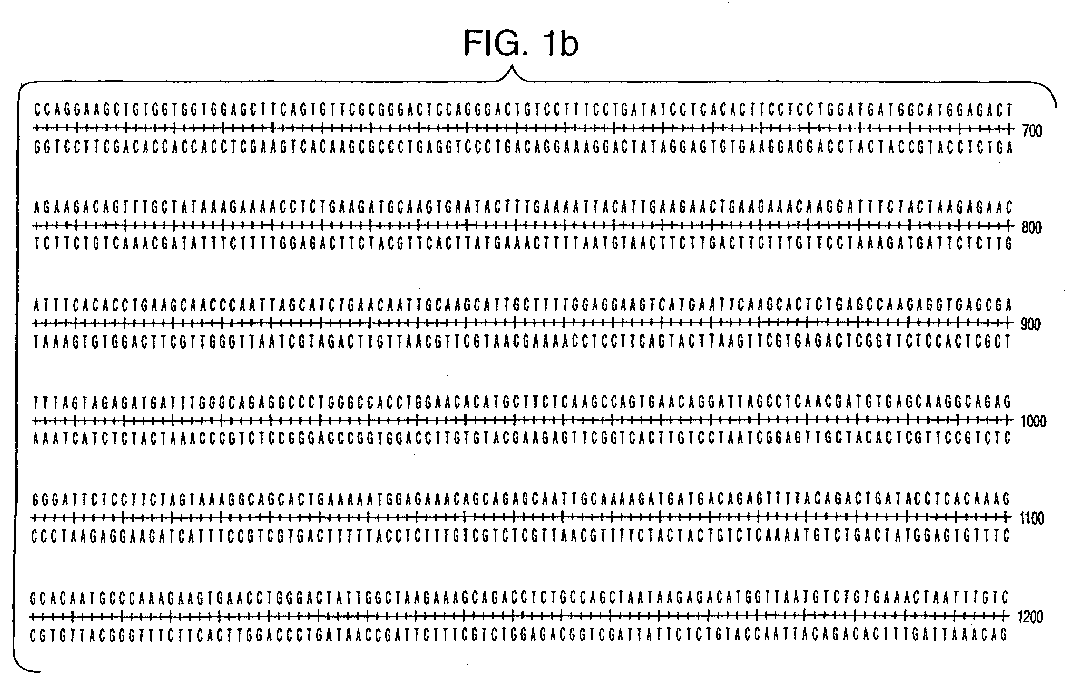

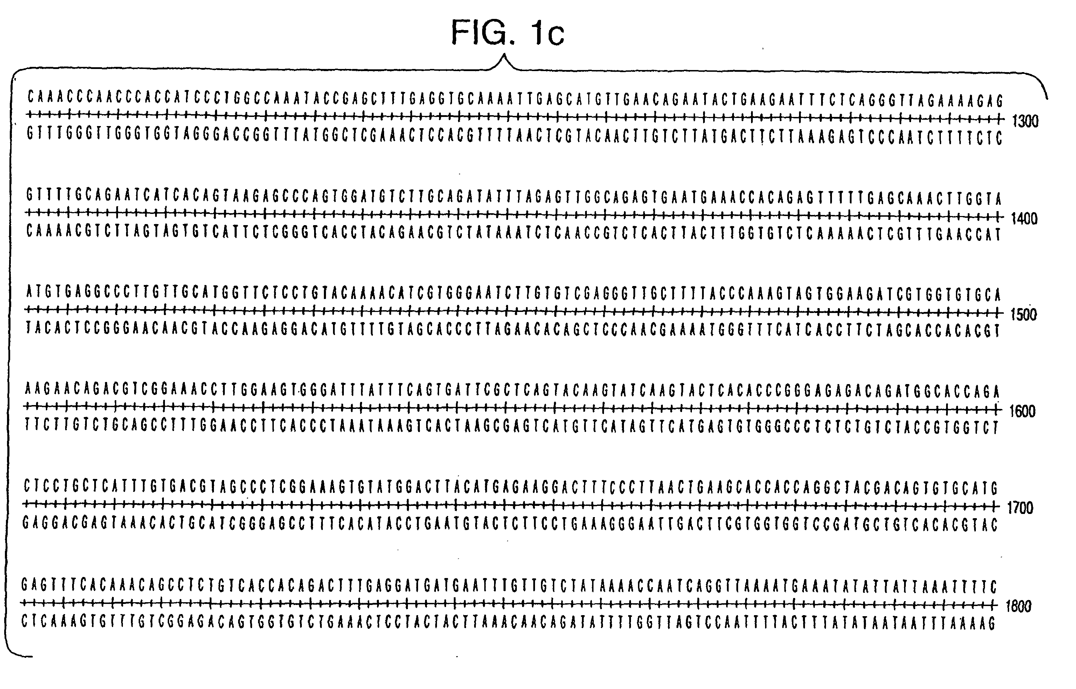

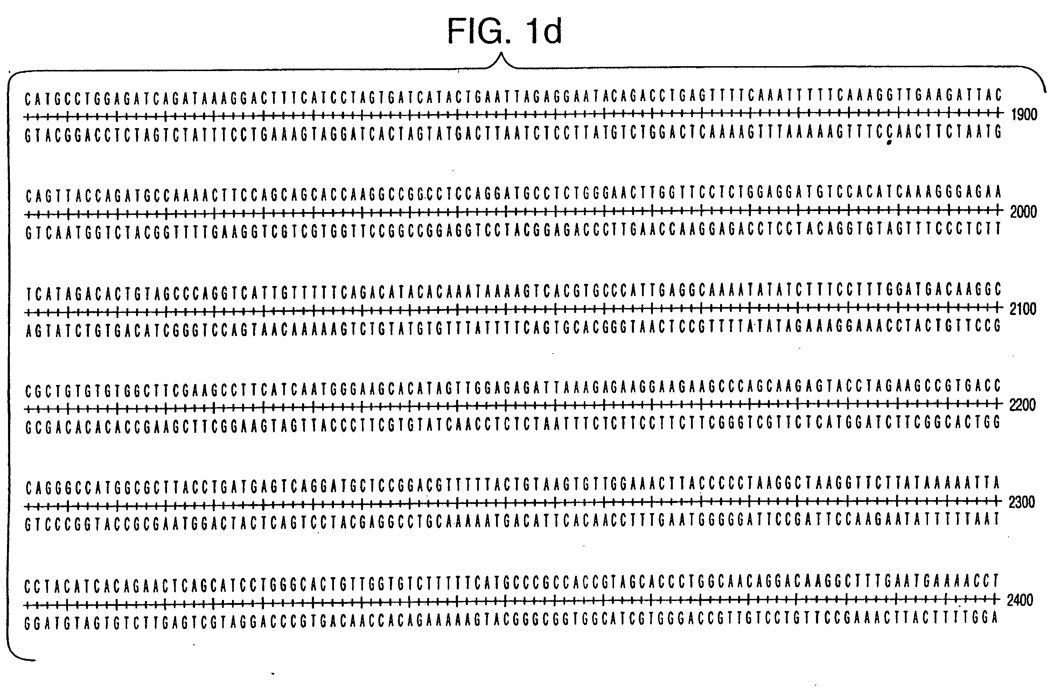

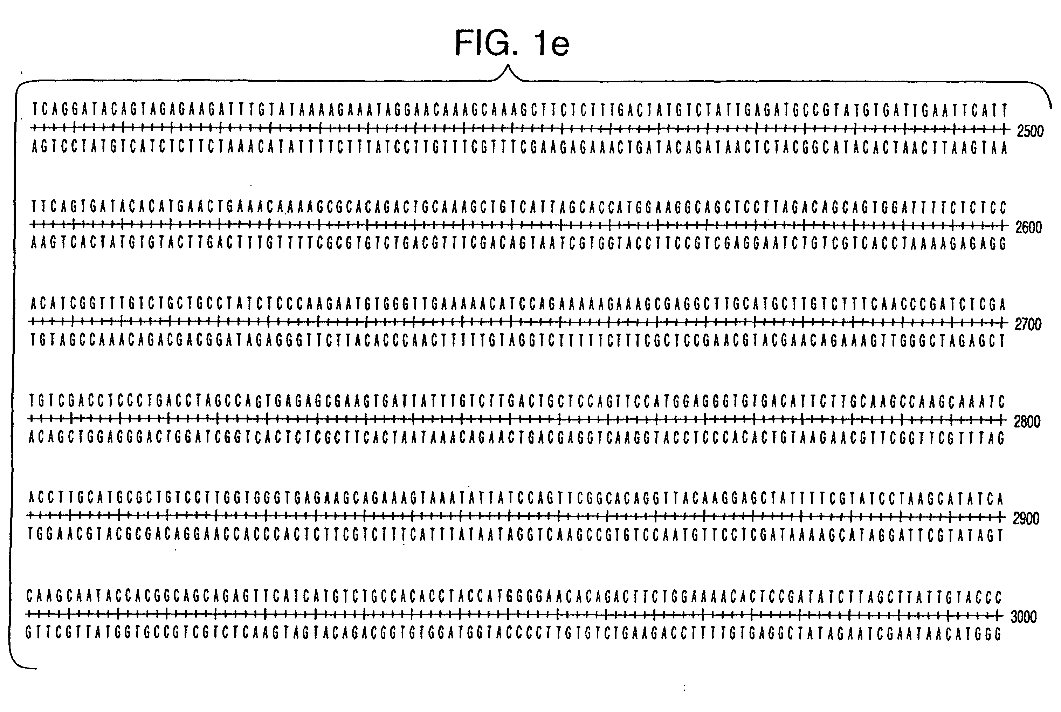

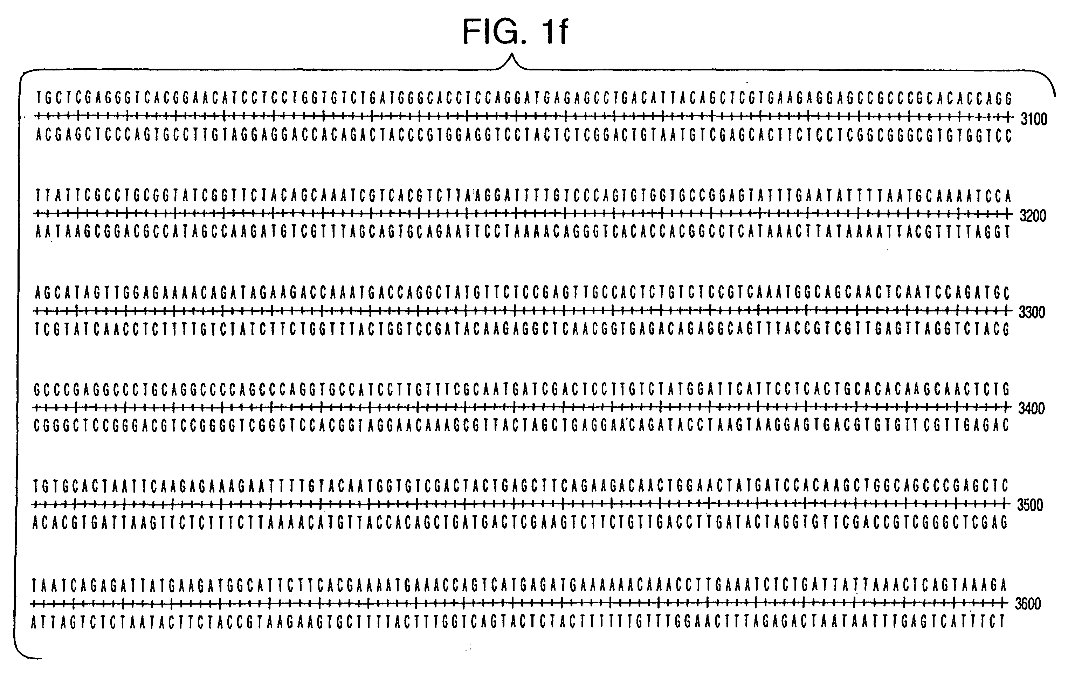

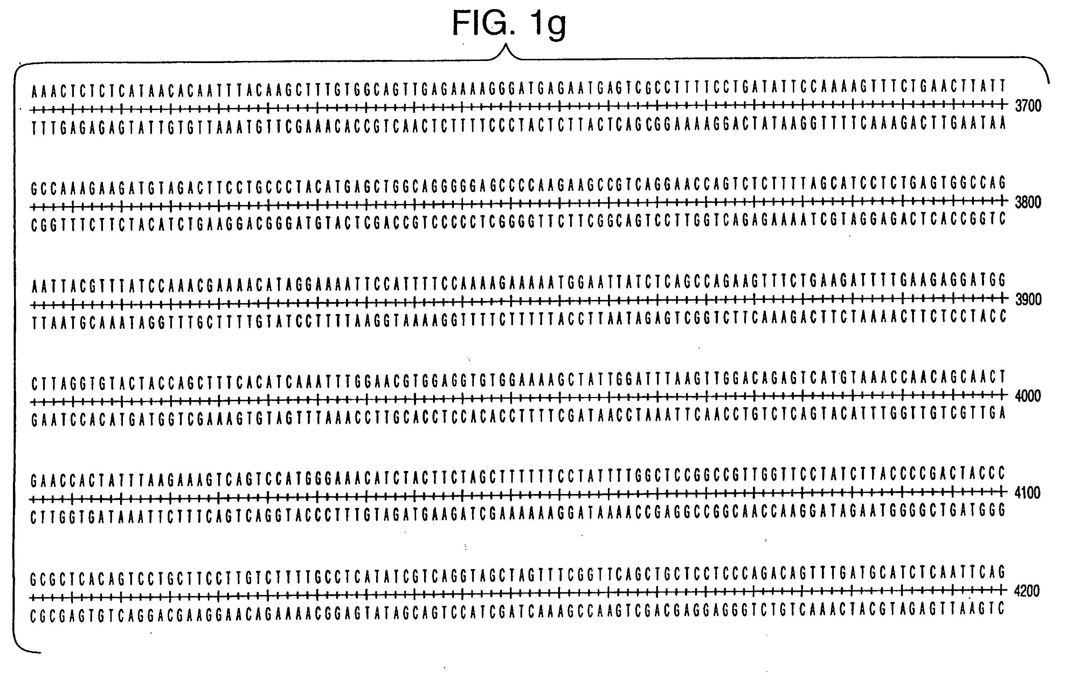

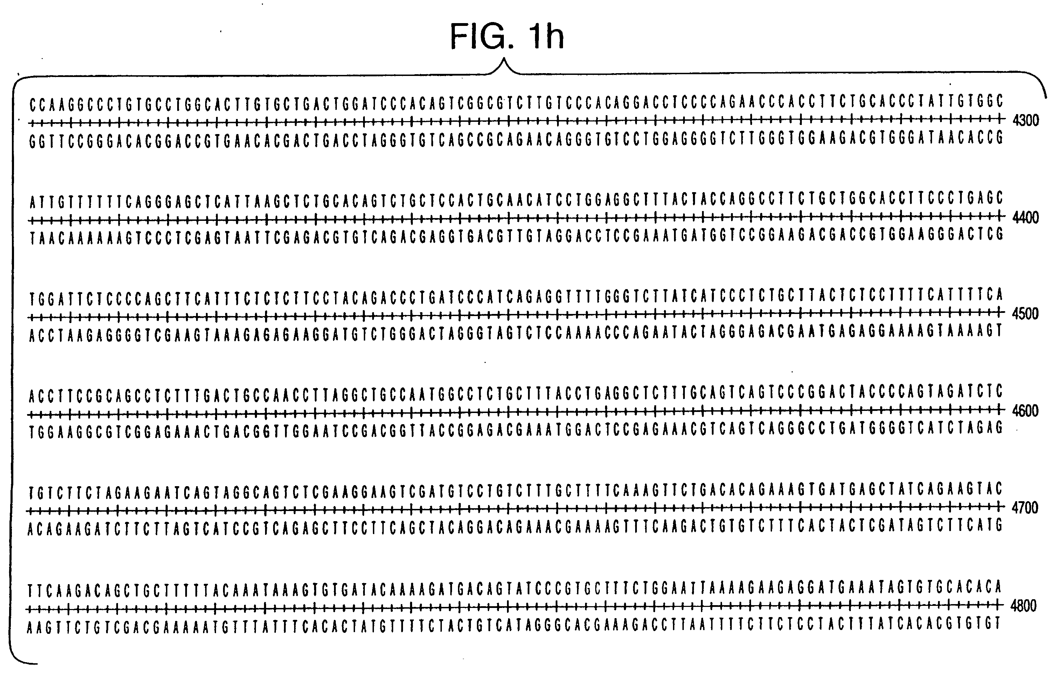

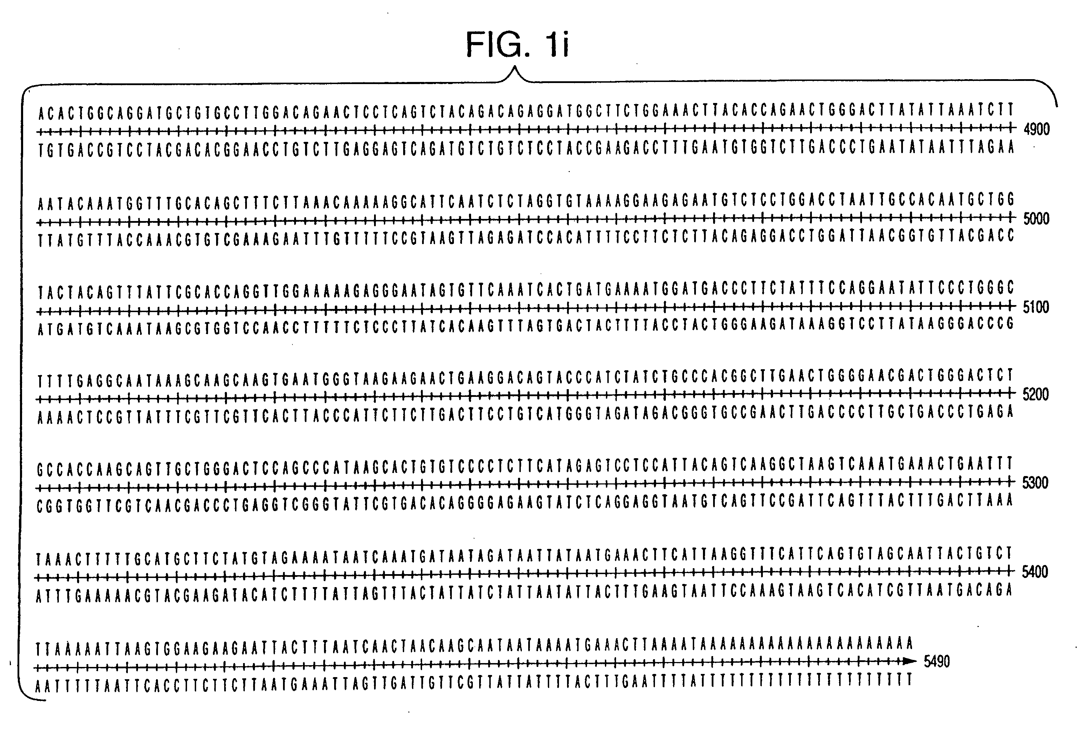

Referring now to Figure 1, there is shown the complete sequence of cDNA encoding human minor vault protein p193, top strand, SEQ ID NO:1, and its complementary strand. As can be seen, the DNA encoding human minor vault protein p193 contains 5490 base pairs. The open reading frame is from residue 107 to residue 5281.

The cDNA encoding human minor vault protein p193 was then used to deduce the amino acid sequence of the human minor vault protein p193, SEQ ID NO:2. Further, human minor vault protein p193 was purified from vaults by electrophoresis on 5% SDS-polyacrylamide gels. The gels were stained with copper (BioRad Laboratories, Hercules, CA, USA) and the identified band was excised and destained, and the amino acids sequenced according to standard techniques using a Finnigan TSQ-7000 Triple Quadrupole Mass Spectrometer. This sequence is the same as SEQ ID NO:2.

Referring now to Figure 2, there is shown the complete amino acid sequence of human minor vault protein p193, SEQ ID NO:2. As can be seen, the sequence includes 1724 amino acid residues.

A search of the National Center for Biotechnology databases was performed to determine if either SEQ ID NO: or SEQ ID NO:2 were previously known. The search revealed a previously known nucleotide sequence, GenBank accession number D79999, having 5085 nucleotides which were identical to residues 384-5469 of SEQ ID NO:1: GenBank accession number D79999 did not, however, include residues 107-383 of SEQ ID NO:1 which constitutes part of the open reading frame.

The search further revealed that residues 209-563 of SEQ ID NO:2 share 28% identity to residues 609-1004, the catalytic subunit of poly (ADP-ribose) polymerase, GenBank accession number M32721, but did not otherwise reveal a homologous sequence. This catalytic subunit binds to NAD, hydrolyzes the nicotine moiety and polymerizes the ADP ribose group.

Analysis of SEQ ID NO:2 using the PROSITE protein database also revealed that residues 1-94 of SEQ ID NO:2 comprise a BRCT domain. BRCT domains refer to the C-terminus of the cancer susceptibility gene BRCA 1, and are a superfamily of conserved domains in DNA damage-response cell cycle checkpoint proteins. See, for example, Bork, et al., The Faseb J. 11:68-76, 1997; and Callebaut, I. and Momon, J-P., FEBS Letter 400:25-30, 1997, incorporated by reference in their entirety.

Referring again to Figure 2, residues 1-94 of human minor vault protein p193, which comprise the BRCT domain, are indicated by the unshaded box. Residues 209-563 of human minor vault protein p193, which share 28% identity to the catalytic subunit of poly (ADP-ribose) polymerase are shown in the upper shaded box. Finally, residues 1562-1724 of human minor vault protein p193, which comprise the region necessary for interaction with human major vault protein, are shown in the lower shaded box.

### (2) Generation of Antibodies to Human Minor Vault Protein p193:

Antibodies which immunoreact with human minor vault protein p193 were produced as follows. First, fragments of human minor vault protein p193 were produced as follows. First, fragments of human minor vault protein p193 were generated using PCR techniques. The fragments consisted of residues 408-611 and residues 1471-1724 of SEQ ID NO:2. Next, fusion proteins were generated and both polyclonal and monoclonal antibodies were produced. These antibodies recognized human minor vault protein p193 in western blots, by immunofluorescence microscopy and by immunoprecipitation.

### (3) Description of Certain Embodiments of the Present Invention:

Therefore, according to the present invention, there is provided a protein consisting essentially of purified human minor vault protein p193, SEQ ID NO:2. The protein can also consist of purified biologically active variants of human minor vault protein p193 or a combination of purified human minor vault protein p193, SEQ ID NO:2, and biologically active variants of human minor vault protein p193. In a preferred embodiment, the protein is a recombinant protein. Further, the present invention includes a protein having an amino acid sequence as set forth in SEQ ID NO:2, as well as a protein recognized by a monoclonal or polyclonal antibody having affinity to a protein according to the present invention.

The protein according to the present invention can be made according to techniques known to those with skill in the art, for example, by first culturing a microorganism transformed with a polynucleotide encoding human minor vault protein p193. Then, the human minor vault protein p193 is recovered from the mircroorganism.

The present invention also includes a polynucleotide molecule encoding a protein which consists essentially of human minor vault protein p193, SEQ ID NO:2, and includes its complementary strands and a polynucleotide molecule which hybridizes thereto. The polynucleotide can be an RNA molecule or a DNA molecule, as well as other polynucleotide molecules.

According to another embodiment of the present invention, there is provided a vector containing a polynucleotide according to the present invention. The vector, such as PET 28 (available from Invitrogen, Carlsbad, CA, USA), pGEX and pSVL (both available from Amersham Pharmacia Biotech, Piscataway, NJ, USA), can be used to stably transform or transfect a prokaryotic or eukaryotic host cell.

The present invention further includes an antibody which immunoreacts with a protein or polynucleotide according to the present invention. The Fc portion of the antibody can be selected from the group consisting of the IgM class, the IgG class and the IgA class, but can also be other classes. Preferably, the antibody is a high affinity monoclonal antibody which immunoreacts with human minor vault protein p193.

The antibody can be made, for example, by administering human minor vault protein p 193 to a host in an amount sufficient to induce the production of antibodies to the human minor vault protein p193 from the antibody-producing cells. Next, the antibody-producing cells are recovered from the host and cell hybrids are formed by fusing the antibody-producing cell to cells capable of substantially unlimited reproduction. Then, the hybrids are cultured and the monoclonal antibodies are collected as a product of the hybrids. Preferably, the cells capable of substantially unlimited reproduction are myeloma cells.

### EXAMPLE I

### METHOD OF DIAGNOSING A PATIENT WITH A MULTIDRUG-RESISTANT CANCER

According to one embodiment of the present invention, a patient with a multidrug-resistant cancer is diagnosed by, first, providing a sample of tissue or fluid from the patient. The sample can be bone marrow, cerebral spinal fluid, blood, tears, saliva or a biopsy specimen, or can be other suitable tissue or fluid samples. Next, the level of a substance selected from the group consisting of p193 protein, p193 DNA, p193 mRNA, a substantial portion of p193 protein, a substantial portion of p193 DNA, a substantial portion of p193 mRNA and a combination of one of the foregoing in the patient sample is determined. In a preferred embodiment, the substantial portion comprises at least about 25 % of the residues of the molecule. In a particularly preferred embodiment, the substantial portion comprises at least about 50% of the residues of the molecule. Then, the level of the substance is compared to a known range of levels for the substance in patients with multidrug-resistant cancers. A diagnosis of multidrug-resistant cancer is made when the level of the substance determined is within the range of levels for the substance in patients with multidrug-resistant cancers.

### EXAMPLE II

### METHOD OF TREATING A PATIENT WITH MULTIDRUG-RESISTANT CANCER

According to another embodiment of the present invention, a patient with a multidrug-resistant cancer is treated by disrupting the production or function of human minor vault protein p193. This is accomplished by, for example, administering to the patient antibodies having an affinity for a substance selected from the group consisting of p193 protein and a polynucleotide encoding p193. Treatment can also be accomplished by administering to the patient at least one antisense polynucleotide having an affinity for a polynucleotide encoding p193. Further, treatment can be accomplished by administering to the patient at least one drug that blocks NAD, such as PD 128763 and 3-aminobenzamide.

Although the present invention has been discussed in considerable detail with reference to certain preferred embodiments, other embodiments are possible. Therefore, the spirit and scope of the appended claims should not be limited to the description of preferred embodiments contained in this application.

## Claims

1. A polynucleotide molecule according to SEQ ID NO:1, or its complementary strands.

2. A vector containing the polynucleotide of claim 1.

3. A prokaryotic or eukaryotic host cell stably transformed or transfected by the vector of claim 2.

4. A method of making a composition. which comprises isolating the polynucleotide molecule of claim 1 and forming the composition therewith.

5. A composition comprising the polynucleotide molecule according to claim 1.

6. A method of making human minor vault protein p193, comprising:
(a) culturing a microorganism transformed with a polynucleotide according to claim 1; and
(b) recovering the human minor vault protein p193.

7. A purified, isolated or purified and isolated protein comprising an amino acid sequence as set forth in SEQ ID NO: 2.

8. A method of making a composition which comprises purifying or isolating an amino acid sequence as set forth in SEQ ID NO:2 and forming the composition therewith.

9. A recombinant protein according to claim 7.

10. A method of making a monoclonal antibody which immunoreacts with human minor vault protein p193 comprising:
(a) administering a protein according to claim 7 to a non-human host in an amount sufficient to induce the production of antibodies to the protein from the antibody-producing cells;
(b) recovering the antibody-producing cells from the host;
(c) forming cell hybrids by fusing the antibody-producing cell to cells capable of substantially unlimited reproduction;
(d) culturing the hybrids; and
(e) collecting the monoclonal antibodies as a product of the hybrids.

11. The method of claim 10, wherein the cells capable of substantially unlimited reproduction in step (c) are myeloma cells.

12. A method of making the protein- according to claim 7, comprising:
culturing a microorganism transformed with a polynucleotide encoding the human minor vault protein p193 having the sequence of SEQ ID NO. 2; and
recovering the human minor vault protein p193.

13. A method of diagnosing a patient with a multidrug-resistant cancer comprising:
(a) determining in a sample the level of a substance selected from the group consisting of a polynucleotide molecule according claim 1, a protein according to claim 7, a substantial portion of a polynucleotide molecule according claim 1, a substantial portion of a protein according to claim 7, and a combination of one of the foregoing in the patient sample; and
(b) comparing the level of the substance determined in step (a) to a known range of levels for the substance in patients with multidrug-resistant cancers, wherein a diagnosis of multidrug-resistant cancer is made when the level of the substance determined in step (a) is within the range of levels for the substance in patients with multidrug-resistant cancers.

14. The method of claim 13, where the sample is selected from the group consisting of bone marrow, cerebral spinal fluid, blood, tears, saliva and a biopsy specimen.

15. Use of antibodies having an affinity for a protein according to claim 7 in the manufacture of a composition for the treatment of a patient with multidrug-resistant cancer.

16. Use of at least one antisense polynucleotide having an affinity for a polynucleotide according to claim 1 in the manufacture of a composition for the treatment of a patient with multidrug-resistant cancer.

17. Use of at least one drug that blocks NAD in combination with antibodies having an affinity for a protein according to claim 7 in the manufacture. of a composition for the treatment of a patient with multidrug-resistant cancer.

18. The use of claim 17, where the drug is selected from the group consisting of PD128763 and 3-aminobenzamide.

19. A method of catalyzing the hydrolysis of the nicotine moiety or of catalyzing the polymerisation of the ADP ribose group of at least one NAD molecule comprising contacting the NAD molecule with the protein according to claim 7.

## Patentansprüche

1. Polynukleotidmolekül gemäß SEQ ID No: 1 oder dessen komplementäre Stränge.

2. Vektor, der das Polynukleotid nach Anspruch 1 enthält.

3. Prokaryotische oder eukaryotische Wirtszelle, die stabil mit dem Vektor nach Anspruch 2 transformiert oder transfiziert ist.

4. Verfahren zur Herstellung einer Zusammensetzung, das das Isolieren des Polynukleotidmoleküls nach Anspruch 1 und das Bilden der Zusammensetzung damit umfasst.

5. Zusammensetzung, die das Polynukleotidmolekül nach Anspruch 1 umfasst.

6. Verfahrung zur Herstellung von humanem Minor Vault Protein p 193, umfassend:
(a) Kultivieren eines Mikroorganismus, der mit einem Polynukleotid nach Anspruch 1 transformiert ist; und
(b) Wiedergewinnen des humanen Minor Vault Protein p193.

7. Aufgereinigtes, isoliertes oder aufgereinigtes und isoliertes Protein, umfassend eine Aminosäuresequenz, wie sie in SEQ ID No:2 dargelegt ist.

8. Verfahren zur Herstellung einer Zusammensetzung, das das Aufreinigen oder Isolieren einer Aminosäuresequenz, wie sie in SEQ ID No:2 dargelegt ist, und das Bilden der Zusammensetzung damit umfasst.

9. Rekombinantes Protein nach Anspruch 7.

10. Verfahren zur Herstellung eines monoklonalen Antikörpers, der mit humanem Minor Vault Protein p193 immunreagiert, umfassend:
(a) Verabreichen eines Proteins nach Anspruch 7 an einen nicht-humanen Wirt in einer Menge, die ausreicht, die Produktion von Antikörpern gegen das Protein durch die Antikörper-produzierenden Zellen zu induzieren;
(b) Wiedergewinnen der Antikörper-produzierenden Zellen aus dem Wirt;
(c) Bilden von Zellhybriden durch Fusionieren der Antikörper-produzierenden Zelle mit zur im wesentlichen unbegrenzten Reproduktion fähigen Zellen;
(d) Kultivieren der Hybride; und
(e) Gewinnen der monoklonalen Antikörper als ein Produkt der Hybride.

11. Verfahren nach Anspruch 10, bei dem die zur im wesentlichen unbegrenzten Reproduktion fähigen Zellen in Schritt (c) Myelomzellen sind.

12. Verfahren zur Herstellung des Proteins nach Anspruch 7, umfassend:
Kultivieren eines Mikroorganismus, der mit einem Polynukleotid, transformiert ist, das für das humane Minor Vault Protein p193, das die Sequenz der SEQ ID NR:2 hat, kodiert, und Wiedergewinnen des humanen Minor Vault Proteins p193.

13. Verfahren zum Diagnostizieren eines Patienten mit Multidrug-resistentem Krebs, umfassend:
(a) Bestimmen des Niveaus einer Substanz in einer Probe, wobei die Substanz ausgewählt ist aus der Gruppe bestehend aus einem Polynukleotidmolekül nach Anspruch 1, einem Protein nach Anspruch 7, einem wesentlichen Teil eines Polynukleotidmoleküls nach Anspruch 1, einem wesentlichen Teil eines Proteins nach Anspruch 7 und einer Kombination aus einem der vorhergehenden in der Patientenprobe und
(b) Vergleichen des in Schritt (a) bestimmten Niveaus der Substanz mit einem bekannten Bereich von Niveaus der Substanz bei Patienten mit Multidrug-resistenten Krebserkrankungen, wobei eine Diagnose von Multidrug-resistentem Krebs erfolgt, wenn das in Schritt (a) bestimmte Niveau der Substanz in einem Bereich von Niveaus der Substanz bei Patienten mit Multidrug-resistenten Krebserkrankungen liegt.

14. Verfahren nach Anspruch 13, bei dem die Probe aus der Gruppe bestehend aus Knochenmark, Cerebralspinalflüssigkeit, Blut, Tränen, Speichel und einer Biopsieprobe ausgewählt ist.

15. Verwendung von Antikörpern, die eine Affinität zu einem Protein nach Anspruch 7 haben, zur Herstellung einer Zusammensetzung zur Behandlung eines Patienten mit Multidrug-resistentem Krebs.

16. Verwendung von mindestens einem Antisinn-Polynukleotid (antisense Polynukleotid), das eine Affinität zu einem Polynukleotid nach Anspruch 1 hat, zur Herstellung einer Zusammensetzung zur Behandlung eines Patienten mit Multidrug-resistentem Krebs.

17. Verwendung von mindestens einem Wirkstoff, der NAD blockiert in Kombination mit Antikörpern, die eine Affinität zu einem Protein nach Anspruch 7 haben, zur Herstellung einer Zusammensetzung zur Behandlung eines Patienten mit Multidrug-resistentem Krebs.

18. Verwendung nach Anspruch 17, bei der der Wirkstoff aus der Gruppe bestehend aus PD 128763 und 3-Aminobenzamid ausgewählt ist.

19. Verfahren zum Katalysieren der Hydrolyse des Nikotinanteils oder zum Katalysieren der Polymerisation der ADP-Ribosegruppe von mindestens einem NAD-Molekül, das das Kontaktieren des NAD-Moleküls mit dem Protein nach Anspruch 7 umfasst.

## Revendications

1. Molécule de polynucléotide selon SEQ ID NO:1, ou ses brins complémentaires.

2. Vecteur contenant le polynucléotide de la revendication 1.

3. Cellule hôte procaryote ou eucaryote transformée ou transfectée de façon stable par le vecteur de la revendication 2.

4. Procédé de fabrication d'une composition qui comprend l'isolement de la molécule de polynucléotide de la revendication 1 et la formation de la composition avec celle-ci.

5. Composition comprenant la molécule de polynucléotide telle que définie à la revendication 1.

6. Procédé de fabrication de la protéine voûte mineure humaine p193, comprenant les opérations consistant à :
(a) cultiver un microorganisme transformé par un polynucléotide tel que défini à la revendication 1 ;
et
(b) récupérer la protéine voûte mineure humaine p193.

7. Protéine purifiée, isolée ou purifiée et isolée, comprenant une séquence d'acides aminés telle qu'exposée en SEQ ID NO:2.

8. Procédé de fabrication d'une composition qui comprend la purification ou l'isolement d'une séquence d'acides aminés telle qu'exposée en SEQ ID NO:2 et la formation de la composition avec celle-ci.

9. Protéine recombinante selon la revendication 7.

10. Procédé de fabrication d'un anticorps monoclonal qui est immunoréactif avec la protéine voûte mineure humaine p193, comprenant les opérations consistant à :
(a) administrer une protéine telle que définie à la revendication 7 à un hôte non-humain dans une quantité suffisante pour induire la production d'anticorps dirigés contre la protéine par les cellules productrices d'anticorps ;
(b) récupérer les cellules productrices d'anticorps à partir de l'hôte ;
(c) former des cellules hybrides par fusion des cellules productrices d'anticorps à des cellules capables d'une reproduction sensiblement illimitée ;
(d) cultiver les hybrides ; et
(e) recueillir les anticorps monoclonaux en tant que produit des hybrides.

11. Procédé selon la revendication 10, dans lequel les cellules capables de reproduction sensiblement illimitée à l'étape (c) sont des cellules de myélome.

12. Procédé de fabrication de la protéine telle que définie à la revendication 7, comprenant les opérations consistant à :
- cultiver un microorganisme transformé par un polynucléotide codant pour la protéine voûte mineure humaine p193 ayant la séquence de SED ID NO:2 ;
- récupérer la protéine voûte mineure humaine p193.

13. Procédé de diagnostic d'un patient présentant un cancer résistant à l'action de plusieurs médicaments, comprenant les opérations consistant à :
(a) déterminer dans un échantillon le taux d'une substance choisie dans le groupe constitué par une molécule de polynucléotide telle que définie à la revendication 1, une protéine telle que définie à la revendication 7, une partie substantielle d'une molécule de polynucléotide selon la revendication 1, une partie substantielle d'une protéine telle que définie à la revendication 7 et une combinaison de l'une des précédentes dans l'échantillon du patient ; et
(b) comparer le taux de la substance déterminée à l'étape
(a) à une plage connue de taux pour la substance dans des patients présentant des cancers résistant à l'action de plusieurs médicaments, un diagnostic de cancer résistant à l'action de plusieurs médicaments étant réalisé lorsque le taux de la substance déterminée à l'étape (a) se trouve dans la plage de taux pour la substance dans des patients présentant des cancers résistant à l'action de plusieurs médicaments.

14. Procédé selon la revendication 13, où l'échantillon est choisi dans le groupe constitué par la moelle osseuse, le fluide spinal cérébral, le sang, les larmes, la salive et un échantillon de biopsie.

15. Utilisation d'anticorps ayant une affinité pour une protéine telle que définie à la revendication 7 dans la fabrication d'une composition pour le traitement d'un patient présentant un cancer résistant à l'action de plusieurs médicaments.

16. Utilisation d'au moins un polynucléotide antisens ayant une affinité pour un polynucléotide tel que défini à la revendication 1 dans la fabrication d'une composition pour le traitement d'un patient présentant un cancer résistant à l'action de plusieurs médicaments.

17. Utilisation d'au moins un médicament qui bloque le NAD en combinaison avec des anticorps ayant une affinité pour une protéine telle que définie à la revendication 7 dans la fabrication d'une composition pour le traitement d'un patient présentant un cancer résistant à l'action de plusieurs médicaments.

18. Utilisation selon la revendication 17, où le médicament est choisi dans le groupe constitué par PD128763 et le 3-aminobenzamide.

19. Procédé de catalyse de l'hydrolyse de la fraction nicotine ou de catalyse de la polymérisation du groupe ribose de l'ADP d'au moins une molécule de NAD, comprenant la mise en contact de la molécule de NAD avec la protéine telle que définie à la revendication 7.


### SEQUENCE LISTING

(1) GENERAL INFORMATION:
   (i) APPLICANT: Rome, Leonard H.
      Kickhoefer, Valerie A.
   (ii) TITLE OF INVENTION: HUMAN MINOR VAULT PROTEIN p193
   (iii) NUMBER OF SEQUENCES: 2
   (iv) CORRESPONDENCE ADDRESS:
      (A) ADDRESSEE: Sheldon & Mak
      (B) STREET: 225 S. Lake Avenue, 9th Floor
      (C) CITY: Pasadena
      (D) STATE: California
      (E) ZIP: 91101
   (v) COMPUTER READABLE FORM:
      (A) MEDIUM TYPE: Diskette, 3.50 inch, 1.44 Mb storage
      (B) COMPUTER: IBM compatible
      (C) OPERATING SYSTEM: Windows 95
      (D) SOFTWARE: WordPerfect for Windows version 8.0
   (vi) CURRENT APPLICATION DATA:
      (A) APPLICATION NUMBER: to be assigned
      (B) FILING DATE: filed herewith
      (C) CLASSIFICATION: to be assigned
   (viii) ATTORNEY/AGENT INFORMATION:
      (A) NAME: Farah, David A.
      (B) REGISTRATION NUMBER: 38,134
      (C) REFERENCE/DOCKET NUMBER: 12401PCT
   (ix) TELECOMMUNICATION INFORMATION:
      (A) TELEPHONE: (626) 796-4000
      (B) TELEFAX: (626) 795-6321
(2) INFORMATION FOR SEQ ID NO:
   (i) SEQUENCE CHARACTERISTICS:
      (A) LENGTH: 5490 base pairs
      (B) TYPE: nucleic acid
      (C) STRANDEDNESS: double stranded
      (D) TOPOLOGY: linear
   (ii) MOLECULE TYPE: cDNA
   (ix) SEQUENCE DESCRIPTION: SEQ ID NO:1:
(2) INFORMATION FOR SEQ ID NO:2:
   (i) SEQUENCE CHARACTERISTICS:
      (A) LENGTH: 1724 amino acids
      (B) TYPE: amino acid
      (C) TOPOLOGY: linear
   (ii) MOLECULE TYPE: protein
   (ix) SEQUENCE DESCRIPTION: SEQ ID NO:2:
   Met Val Met Gly Ile Phe Ala Asn Cys Ile Phe Cys Leu Lys Val Lys Tyr Leu
   1 5 10 15
   Pro Gln Gln Gln Lys Lys Lys Leu Gln Thr Asp Ile Lys Glu Asn Gly Gly Lys
   20 25 30 35
   Phe Ser Phe Ser Leu Asn Pro Gln Cys Thr His Ile Ile Leu Asp Asn Ala Asp
   40 45 50
   Val Leu Ser Gln Tyr Gln Leu Asn Ser Ile Gln Lys Asn His Val His Ile Ala
   55 60 65 70
   Asn Pro Asp Phe Ile Trp Lys Ser Ile Arg Glu Lys Arg Leu Leu Asp Val Lys
   75 80 85 90
   Asn Tyr Asp Pro Tyr Lys Pro Leu Asp Ile Thr Pro Pro Pro Asp Gln Lys Ala
   95 100 105
   Ser Ser Ser Glu Val Lys Thr Glu Gly Leu Cys Pro Asp Ser Ala Thr Glu Glu
   110 115 120 125
   Glu Asp Thr Val Glu Leu Thr Glu Phe Gly Met Gln Asn Val Glu Ile Phe His
   130 135 140
   Leu Pro Gln Asp Phe Glu Val Ala Lys Tyr Asn Thr Leu Glu Lys Val Gly Met
   145 150 155 160
   Glu Gly Gly Gln Glu Ala Val Val Val Glu Leu Gln Cys Ser Arg Asp Ser Arg
   165 170 175 180
   Asp Cys Pro Phe Leu Ile Ser Ser His Phe Leu Leu Asp Asp Gly Met Glu Thr
   185 190 195
   Arg Arg Gln Phe Ala Ile Lys Lys Thr Ser Glu Asp Ala Ser Glu Tyr Phe Glu
   200 205 210 215
   Asn Tyr Ile Glu Glu Leu Lys Lys Gln Gly Phe Leu Leu Arg Glu His Phe Thr
   220 225 230
   Pro Glu Ala Thr Gln Leu Ala Ser Glu Gln Leu Gln Ala Leu Leu Leu Glu Glu
   235 240 245 250
   Val Met Asn Ser Ser Thr Leu Ser Gln Glu Val Ser Asp Leu Val Glu Met Ile
   255 260 265 270
   Trp Ala Glu Ala Leu Gly His Leu Glu His Met Leu Leu Lys Pro Val Asn Arg
   275 280 285
   Ile Ser Leu Asn Asp Val Ser Lys Ala Glu Gly Ile Leu Leu Leu Val Lys Ala
   290 295 300 305
   Ala Leu Lys Asn Gly Glu Thr Ala Glu Gln Leu Gln Lys Met Met Thr Glu Phe
   310 315 320
   Tyr Arg Leu Ile Pro His Lys Gly Thr Met Pro Lys Glu Val Asn Leu Gly Leu
   325 330 335 340
   Leu Ala Lys Lys Ala Asp Leu Cys Gln Leu Ile Arg Asp Met Val Asn Val Cys
   345 350 355 360
   Glu Thr Asn Leu Ser Lys Pro Asn Pro Pro Ser Leu Ala Lys Tyr Arg Ala Leu
   365 370 375
   Arg Cys Lys Ile Glu His Val Glu Gln Asn Thr Glu Glu Phe Leu Arg Val Arg
   380 385 390 395
   Lys Glu Val Leu Gln Asn His His Ser Lys Ser Pro Val Asp Val Leu Gln Ile
   400 405 410
   Phe Arg Val Gly Arg Val Asn Glu Thr Thr Glu Phe Leu Ser Lys Leu Gly Asn
   415 420 425 430
   Val Arg Pro Leu Leu His Gly Ser Pro Val Gln Asn Ile Val Gly Ile Leu Cys
   435 440 445 450
   Arg Gly Leu Leu Leu Pro Lys Val Val Glu Asp Arg Gly Val Gln Arg Thr Asp
   455 460 465
   Val Gly Asn Leu Gly Ser Gly Ile Tyr Phe Ser Asp Ser Leu Ser Thr Ser Ile
   470 475 480 485
   Lys Tyr Ser His Pro Gly Glu Thr Asp Gly Thr Arg Leu Leu Leu Ile Cys Asp
   490 495 500
   Val Ala Leu Gly Lys Cys Met Asp Leu His Glu Lys Asp Phe Pro Leu Thr Glu
   505 510 515 520
   Ala Pro Pro Gly Tyr Asp Ser Val His Gly Val Ser Gln Thr Ala Ser Val Thr
   525 530 535 540
   Thr Asp Phe Glu Asp Asp Glu Phe Val Val Tyr Lys Thr Asn Gln Val Lys Met
   545 550 555
   Lys Tyr Ile Ile Lys Phe Ser Met Pro Gly Asp Gln Ile Lys Asp Phe His Pro
   560 565 570 575
   Ser Asp His Thr Glu Leu Glu Glu Tyr Arg Pro Glu Phe Ser Asn Phe Ser Lys
   580 585 590
   Val Glu Asp Tyr Gln Leu Pro Asp Ala Lys Thr Ser Ser Ser Thr Lys Ala Gly
   595 600 605 610
   Leu Gln Asp Ala Ser Gly Asn Leu Val Pro Leu Glu Asp Val His Ile Lys Gly
   615 620 625 630
   Arg Ile Ile Asp Thr Val Ala Gln Val Ile Val Phe Gln Thr Tyr Thr Asn Lys
   635 640 645
   Ser His Val Pro Ile Glu Ala Lys Tyr Ile Phe Pro Leu Asp Asp Lys Ala Ala
   650 655 660 665
   Val Cys Gly Phe Glu Ala Phe Ile Asn Gly Lys His Ile Val Gly Glu Ile Lys
   670 675 680
   Glu Lys Glu Glu Ala Gln Gln Glu Tyr Leu Glu Ala Val Thr Gln Gly His Gly
   685 690 695 700
   Ala Tyr Leu Met Ser Gln Asp Ala Pro Asp Val Phe Thr Val Ser Val Gly Asn
   705 710 715 720
   Leu Pro Pro Lys Ala Lys Val Leu Ile Lys Ile Thr Tyr Ile Thr Glu Leu Ser
   725 730 735
   Ile Leu Gly Thr Val Gly Val Phe Phe Met Pro Ala Thr Val Ala Pro Trp Gln
   740 745 750 755
   Gln Asp Lys Ala Leu Asn Glu Asn Leu Gln Asp Thr Val Glu Lys Ile Cys Ile
   760 765 770
   Lys Glu Ile Gly Thr Lys Gln Ser Phe Ser Leu Thr Met Ser Ile Glu Met Pro
   775 780 785 790
   Tyr Val Ile Glu Phe Ile Phe Ser Asp Thr His Glu Leu Lys Gln Lys Arg Thr
   795 800 805 810
   Asp Cys Lys Ala Val Ile Ser Thr Met Glu Gly Ser Ser Leu Asp Ser Ser Gly
   815 820 825
   Phe Ser Leu His Ile Gly Leu Ser Ala Ala Tyr Leu Pro Arg Met Trp Val Glu
   830 835 840 845
   Lys His Pro Glu Lys Glu Ser Glu Ala Cys Met Leu Val Phe Gln Pro Asp Leu
   850 855 860
   Asp Val Asp Leu Pro Asp Leu Ala Ser Glu Ser Glu Val Ile Ile Cys Leu Asp
   865 870 875 880
   Cys Ser Ser Ser Met Glu Gly Val Thr Phe Leu Gln Ala Lys Gln Ile Thr Leu
   885 890 895 900
   His Ala Leu Ser Leu Val Gly Glu Lys Gln Lys Val Asn Ile Ile Gln Phe Gly
   905 910 915
   Thr Gly Tyr Lys Glu Leu Phe Ser Tyr Pro Lys His Ile Thr Ser Asn Thr Thr
   920 925 930 935
   Ala Ala Glu Phe Ile Met Ser Ala Thr Pro Thr Met Gly Asn Thr Asp Phe Trp
   940 945 950
   Lys Thr Leu Arg Tyr Leu Ser Leu Leu Tyr Pro Ala Arg Gly Ser Arg Asn Ile
   955 960 965 970
   Leu Leu Val Ser Asp Gly His Leu Gln Asp Glu Ser Leu Thr Leu Gln Leu Val
   975 980 985 990
   Lys Arg Ser Arg Pro His Thr Arg Leu Phe Ala Cys Gly Ile Gly Ser Thr Ala
   995 1000 1005
   Asn Arg His Val Leu Arg Ile Leu Ser Gln Cys Gly Ala Gly Val Phe Glu Tyr
   1010 1015 1020 1025
   Phe Asn Ala Lys Ser Lys His Ser Trp Arg Lys Gln Ile Glu Asp Gln Met Thr
   1030 1035 1040
   Arg Leu Cys Ser Pro Ser Cys His Ser Val Ser Val Lys Trp Gln Gln Leu Asn
   1045 1050 1055 1060
   Pro Asp Ala Pro Glu Ala Leu Gln Ala Pro Ala Gln Val Pro Ser Leu Phe Arg
   1065 1070 1075 1080
   Asn Asp Arg Leu Leu Val Tyr Gly Phe Ile Pro His Cys Thr Gln Ala Thr Leu
   1085 1090 1095
   Cys Ala Leu Ile Gln Glu Lys Glu Phe Cys Thr Met Val Ser Thr Thr Glu Leu
   1100 1105 1110 1115
   Gln Lys Thr Thr Gly Thr Met Ile His Lys Leu Ala Ala Arg Ala Leu Ile Arg
   1120 1125 1130
   Asp Tyr Glu Asp Gly Ile Leu His Glu Asn Glu Thr Ser His Glu Met Lys Lys
   1135 1140 1145 1150
   Gln Thr Leu Lys Ser Leu Ile Ile Lys Leu Ser Lys Glu Asn Ser Leu Ile Thr
   1155 1160 1165 1170
   Gln Phe Thr Ser Phe Val Ala Val Glu Lys Arg Asp Glu Asn Glu Ser Pro Phe
   1175 1180 1185
   Pro Asp Ile Pro Lys Val Ser Glu Leu Ile Ala Lys Glu Asp Val Asp Phe Leu
   1190 1195 1200 1205
   Pro Tyr Met Ser Trp Gln Gly Glu Pro Gln Glu Ala Val Arg Asn Gln Ser Leu
   1210 1215 1220
   Leu Ala Ser Ser Glu Trp Pro Glu Leu Arg Leu Ser Lys Arg Lys His Arg Lys
   1225 1230 1235 1240
   Ile Pro Phe Ser Lys Arg Lys Met Glu Leu Ser Gln Pro Glu Val Ser Glu Asp
   1245 1250 1255 1260
   Phe Glu Glu Asp Gly Leu Gly Val Leu Pro Ala Phe Thr Ser Asn Leu Glu Arg
   1265 1270 1275
   Gly Gly Val Glu Lys Leu Leu Asp Leu Ser Trp Thr Glu Ser Cys Lys Pro Thr
   1280 1285 1290 1295
   Ala Thr Glu Pro Leu Phe Lys Lys Val Ser Pro Trp Glu Thr Ser Thr Ser Ser
   1300 1305 1310
   Phe Phe Pro Ile Leu Ala Pro Ala Val Gly Ser Tyr Leu Thr Pro Thr Thr Arg
   1315 1320 1325 1330
   Ala His Ser Pro Ala Ser Leu Ser Phe Ala Ser Tyr Arg Gln Val Ala Ser Phe
   1335 1340 1345 1350
   Gly Ser Ala Ala Pro Pro Arg Gln Phe Asp Ala Ser Gln Phe Ser Gln Gly Pro
   1355 1360 1365
   Val Pro Gly Thr Cys Ala Asp Trp Ile Pro Gln Ser Ala Ser Cys Pro Thr Gly
   1370 1375 1380 1385
   Pro Pro Gln Asn Pro Pro Ser Ala Pro Tyr Cys Gly Ile Val Phe Ser Gly Ser
   1390 1395 1400
   Ser Leu Ser Ser Ala Gln Ser Ala Pro Leu Gln His Pro Gly Gly Phe Thr Thr
   1405 1410 1415 1420
   Arg Pro Ser Ala Gly Thr Phe Pro Glu Leu Asp Ser Pro Gln Leu His Phe Ser
   1425 1430 1435 1440
   Leu Pro Thr Asp Pro Asp Pro Ile Arg Gly Phe Gly Ser Tyr His Pro Ser Ala
   1445 1450 1455
   Tyr Ser Pro Phe His Phe Gln Pro Ser Ala Ala Ser Leu Thr Ala Asn Leu Arg
   1460 1465 1470 1475
   Leu Pro Met Ala Ser Ala Leu Pro Glu Ala Leu Cys Ser Gln Ser Arg Thr Thr
   1480 1485 1490
   Pro Val Asp Leu Cys Leu Leu Glu Glu Ser Val Gly Ser Leu Glu Gly Ser Arg
   1495 1500 1505 1510
   Cys Pro Val Phe Ala Phe Gln Ser Ser Asp Thr Glu Ser Asp Glu Leu Ser Glu
   1515 1520 1525 1530
   Val Leu Gln Asp Ser Cys Phe Leu Gln Ile Lys Cys Asp Thr Lys Asp Asp Ser
   1535 1540 1545
   Ile Pro Cys Phe Leu Glu Leu Lys Glu Glu Asp Glu Ile Val Cys Thr Gln His
   1550 1555 1560 1565
   Trp Gln Asp Ala Val Pro Trp Thr Glu Leu Leu Ser Leu Gln Thr Glu Asp Gly
   1570 1575 1580
   Phe Trp Lys Leu Thr Pro Glu Leu Gly Leu Ile Leu Asn Leu Asn Thr Asn Gly
   1585 1590 1595 1600
   Leu His Ser Phe Leu Lys Gln Lys Gly Ile Gln Ser Leu Gly Val Lys Gly Arg
   1605 1610 1615 1620
   Glu Cys Leu Leu Asp Leu Ile Ala Thr Met Leu Val Leu Gln Phe Ile Arg Thr
   1625 1630 1635
   Arg Leu Glu Lys Glu Gly Ile Val Phe Lys Ser Leu Met Lys Met Asp Asp Pro
   1640 1645 1650 1655
   Ser Ile Ser Arg Asn Ile Pro Trp Ala Phe Glu Ala Ile Lys Gln Ala Ser Glu
   1660 1665 1670
   Trp Val Arg Arg Thr Glu Gly Gln Tyr Pro Ser Ile Cys Pro Arg Leu Glu Leu
   1675 1680 1685 1690
   Gly Asn Asp Trp Asp Ser Ala Thr Lys Gln Leu Leu Gly Leu Gln Pro Ile Ser
   1695 1700 1705 1710
   Thr Val Ser Pro Leu His Arg Val Leu His Tyr Ser Gln Gly
   1715 1720